(19) Europäisches Patentamt
European Patent Office
Office européen des brevets

(11) Veröffentlichungsnummer: **0 019 581**
**A1**

(12) # EUROPÄISCHE PATENTANMELDUNG

(21) Anmeldenummer: **80810138.0**

(22) Anmeldetag: **25.04.80**

(51) Int. Cl.[3]: **C 07 D 233/61**
A 01 N 43/50, C 07 C 119/16

(30) Priorität: **04.05.79 CH 4208/79**

(43) Veröffentlichungstag der Anmeldung:
**26.11.80 Patentblatt 80/24**

(84) Benannte Vertragsstaaten:
**BE CH DE FR GB IT LI NL**

(71) Anmelder: **CIBA-GEIGY AG**
**Patentabteilung Postfach**
**CH-4002 Basel(CH)**

(72) Erfinder: **Gloor, Bernhard, Dr.**
**Höhenweg 10**
**CH-4133 Pratteln(CH)**

(72) Erfinder: **Vogel, Christian, Dr.**
**Leimgrubenweg 23**
**CH-4102 Binningen(CH)**

(54) **1-Imidazolo-N-(phenyl)-azomethin-Derivate, Verfahren zu ihrer Herstellung, diese Verbindungen enthaltende Mittel und ihre Verwendung zur Bekämpfung von Schädlingen.**

(57) Verbindungen der Formel

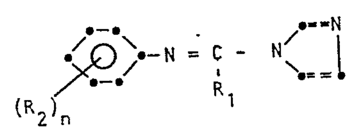

worin n für eine ganze Zahl zwischen 0 und 4 steht, $R_1$ $C_1$-$C_6$-Halogenalkyl, $C_3$-$C_6$ Cycloalkyl oder $C_3$-$C_6$ Halogencycloalkyl bedeutet, und $R_2$ eine durch n bestimmte Zahl gleicher oder verschiedener Substituenten aus der Gruppe Halogen, $C_1$-$C_4$-Alkyl, $C_1$-$C_4$-Alkoxy, Nitro, Trifluormethyl oder Cyano bedeutet.

Die Verbindungen sind für die Schädlingsbekämpfung geeignet und besitzen mikrobizide und akarizide Wirkung. Ferner sind sie als Synergisten zusammen mit bekannten Insektiziden, insbesondere insektiziden Phosphorsäureestern, wirksam.

EP 0 019 581 A1

Croydon Printing Company Ltd.

CIBA-GEIGY AG
Basel (Schweiz)

5-12 337/1+2

BEZEICHNUNG GEÄNDERT
siehe Titelseite

1-Imidazolo-N-(phenyl)-azomethin-Derivate

Die vorliegende Erfindung betrifft neue Verbindungen der Formel I

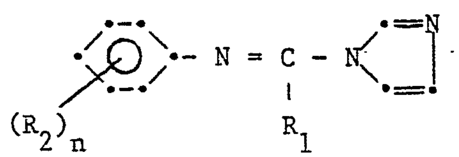

(I)

worin n für eine ganze Zahl zwischen 0 und 4 steht, $R_1$ $C_1$-$C_6$-Halogenalkyl, $C_3$-$C_6$ Cycloalkyl oder $C_3$-$C_6$ Halogencycloalkyl bedeutet, und $R_2$ eine durch n bestimmte Zahl gleicher oder verschiedener Substituenten aus der Gruppe Halogen, $C_1$-$C_4$-Alkyl, $C_1$-$C_4$-Alkoxy, Nitro, Trifluormethyl oder Cyano bedeutet.

Die Erfindung betrifft ferner Schädlingsbekämpfungsmittel, die Verbindungen der Formel I als Wirkstoffe enthalten, Verfahren zur Herstellung dieser Verbindungen und deren Verwendung in der Schädlingsbekämpfung.

Wegen ihrer Wirkung bevorzugt sind erfindungsgemäss Verbindungen der Formel I, die dadurch gekennzeichnet, sind, dass

$R_1$ durch 1 bis 3 Halogenatome substituiertes Methyl, $C_3$-$C_6$-Cycloalykl oder durch 1 Chloratom substituiertes $C_3$-$C_6$-Cycloalkyl und
$R_2$ Chlor, Brom, Methoxy, Methyl, Aethyl oder Trifluormethyl bedeuten.

Hervorzuheben sind auch diejenigen Verbindungen der Formel I, worin

$R_1$ $C_3$-$C_6$-Cycloalkyl oder durch 1 Chloratom substituiertes Cyclohexyl und

$R_2$ Chlor, Methoxy oder Trifluormethyl bedeuten;

sowie diejenigen Verbindungen der Formel I, worin

$R_1$ $C_3$-$C_6$-Cycloalkyl und

$R_2$ Chlor bedeuten.

Eine bevorzugte Gruppe von Verbindungen der Formel I ist dadurch gekennzeichnet, dass n für eine ganze Zahl zwischen 0 und 2, vorzugsweise für 1 oder 2, steht.

Besonders wertvoll sind erfindungsgemässe Verbindungen der Formel I, worin n für 2 steht, $R_1$ Cyclopropyl und $R_2$ Chlor bedeuten.

Unter Alkyl oder als Alkylteil eines anderen Substituenten sind je nach Zahl der angegebenen C-Atome folgende Gruppen zu verstehen: Methyl, Aethyl, Propyl, Butyl, Pentyl oder Hexyl sowie ihre Isomere, wie z.B. iso-Propyl, iso-Butyl, sek.-Butyl, tert.-Butyl, iso-Pentyl usw.

Als Cycloalkyl sind Cyclopropyl, Cyclobutyl, Cyclopentyl und Cyclohexyl zu verstehen.

Halogen steht für Fluor, Chlor, Brom oder Jod.

Aus der deutschen Offenlegungsschrift 2 814 041 und der japanischen offengelegten Patentanmeldung 39 674/77 sind bereits substituierte 1-Imidazolo-N-(phenyl)-azomethin-Derivate als Fungizide bzw. Bakterizide zur Verwendung im Pflanzenschutz bekannt.

Die Verbindungen der Formel I können in an sich bekannter Weise hergestellt werden, indem man eine Verbindung der Formel II

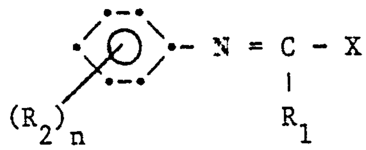

(II)

mit einer Verbindung der Formel III

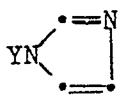

(III)

umsetzt, wobei in den Formeln II und III $R_1$, $R_2$ und n die für Formel I gegebenen Bedeutungen haben, X für ein Nukleofug und Y für Wasserstoff, ein Metallatom oder ein Ammoniumion stehen.

Als Nukleofug kommen in erster Linie der Rest einer anorganischen Säure wie z.B. Halogenid, vorzugsweise Chlorid oder Bromid, oder der einer organischen Sulfonsäure wie Tosyloxy oder Mesyloxy in Frage. Y als Metallatom ist vorteilhafterweise das Atom eines Alkali- oder Erdalkalimetalls wie z.B. Natrium oder Kalium und als Ammoniumion darf es auch substituiert sein. Das Verfahren zur Herstellung der Verbindungen der Formel I bildet ebenfalls einen Teil der Erfindung.

Umsetzungen werden in An- oder Abwesenheit von gegenüber den Reaktionsteilnehmern inerten Lösungsmitteln durchgeführt. Beispiele für geeignete Lösungsmittel sind: aliphatische oder aromatische Kohlenwasserstoffe wie Hexan, Cyclohexan, Benzol, Toluol, Xylol, Petroläther; halogenierte Kohlenwasserstoffe wie Chlorbenzol, Methylenchlorid, Aethylenchlorid, Chloroform, Trichloräthan; Aether oder ätherartige Verbindungen wie Dialkyläther, Tetrahydrofuran, Dioxan; Ketone wie Aceton, Methyläthylketon; Nitrile wie Acetonitril; N,N-Dialkylierte Amide wie Dimethylformamid sowie Gemische solcher Lösungsmittel.

Wenn Y=Wasserstoff ist, wird das Verfahren in Gegenwart eines säurebindenden Mittels durchgeführt. Als säurebindende Mittel kommen beispielsweise folgende Substanzen in Betracht: Basen wie Hydroxide und Carbonate von Alkali- und Erdalkalimetallen wie z.B. Soda,

Pottasche, Aetznatron, Aetzkali; tertiäre Amine wie Triäthylamin, Dimethylanilin, Pyridinbasen; Hydride wie Natrium oder Calciumhydrid, Amide wie Natrium- oder Kaliumamid, Lithiumdiisopropylamid oder aber ein Ueberschuss des 1,2,4-Triazols der Formel III.

Die Reaktionstemperaturen liegen normalerweise zwischen -20° und 120° Celsius vorzugsweise -10° bis 100° Celsius.

Die Ausgangsstoffe der Formel II werden nach an sich bekannten Methoden hergestellt wie z.B. nachfolgend schematisch dargestellt

a) 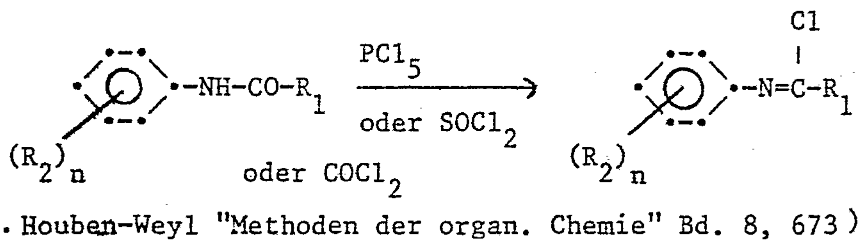

(II)

(vgl. Houben-Weyl "Methoden der organ. Chemie" Bd. 8, 673)

b) Herstellung von II in situ und Weiterreaktion mit III

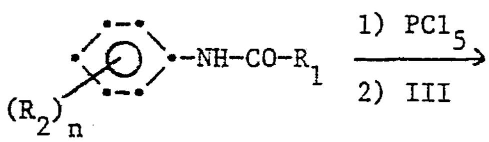

I

(vgl. Kaufmann et al. Chem.Ber. 75, 1585 (1942))

oder 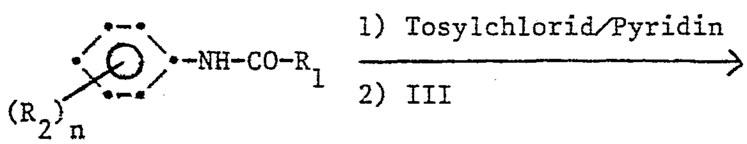

I

(vgl. Oxley et al. JCS, 1948, 1618)

Die Ausgangsstoffe der Formel II sind zum Teil neu und besitzen eine gewisse Fungizide Wirkung. Sie gehören ebenfalls zur Erfindung, desgleichen Schädlingsbekämpfungsmittel, die Verbindungen der Formel II als Wirkstoffe enthalten.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln. Die Herstellung solcher Mittel erfolgt in an sich bekannter Weise durch inniges Vermischen und Vermahlen der Bestandteile. Zur Applikation können die Verbindungen der Formel I in den folgenden Aufarbeitungsformen vorliegen (wobei die Gewichtsprozentangaben vorteilhafte Mengen an Wirkstoff darstellen).

Feste Aufarbeitungsformen: Stäubemittel, Streumittel, (bis 10 %) Granulate, Umhüllungsgranulate, Imprägnierungsgranulate und Imprägnierungsgranulate und Homogengranulate, Pellets (Körner) (1 bis 80 %)

Flüssige Aufarbeitsformen:

a) in Wasser dispergierbare Wirkstoffkonzentrate: Spritzpulver (wettable powders) Pasten, 25-90 % in der Handelspackung, 0,01 bis 15 % in gebrauchsfertiger Lösung, Emulsionen, Lösungskonzentrate (10 bis 50 %); (0,01 bis 15 % in gebrauchsfertiger Lösung)

b) Lösungen: Aerosole:

Der Gehalt an Wirkstoff in den oben beschriebenen Mitteln liegt zwischen 0,1 und 95 Gewichtsprozent. Solche Mittel sind gleichfalls Gegenstand dieser Erfindung.

Die Verbindungen der Formel I besitzen ein für die praktischen Bedürfnisse sehr günstiges Mikrobizid-Spektrum zum Schutz von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, Reben, Hopfen, Gurkengewächse (Gurken, Kürbis, Melonen), Solanaceen wie Kartoffeln, Tabak und Tomaten, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilzen wirksam: Ascomycetes (z.B. Erysiphaceae, Fusarium, Podosphaera, Venturia, Helminthosporium); Basidiomycetes wie vor allem Rostpilze (z.B. Puccinia, Tilletia, Rhizoctonia); Fungi imperfecti und die der Klasse Phycomycetes angehörenden Oomycetes wie Phytophthora und Plasmopara. Die Wirkstoffe sind auch gegen pflanzenschädigende Akarina aktiv. Als Akarina können z.B. Tetranychus urticae und Tetranychus cinnabarius genannt werden, die in allen Stadien (Ei, Larven, Adulte) mit Verbindungen der Formel I bekämpft werden können.

Die Verbindungen sind ferner gegen phytopathogene Bakterien wie Xanthomonas sp., Pseudomonas sp., Erwinia sowie Corynebacterium aktiv.

Die Erfindung betrifft somit ferner die Verwendung der Verbindungen der Formel I zur Bekämpfung von phytopathogenen Mikroorganismen und Vertretern der Ordnung Akarina.

Die Verbindungen der Formel I können, um sie an gegebene Umständen anzupassen, zur Erhöhung der Wirksamkeit und Verbreiterung des Wirkungsspektrums zusammen mit anderen geeigneten Pestiziden, wie z.B. Fungiziden, Bakteriziden, Insektiziden, Akariziden, Herbiziden oder den Pflanzenwuchs beeinflussenden Wirkstoffen eingesetzt werden. So sind die erfindungsgemässen Verbindungen der Formel I auch als Synergisten in Kombination mit anderen bekannten Insektiziden

vom Phosphorsäureester-, Chlorkohlenwasserstoff-, Carbamat- und Pyrethroid-Typ, insbesondere gegenüber resistenten Insektenstämmen, wirksam. Ueberraschenderweise ist die insektizide Wirksamkeit der genannten Wirkstoffkombinationen wesentlich höher als die Summe der Wirkungen der einzelnen Wirkstoffe. Es liegt demnach ein echter synergistischer Effekt vor und nicht nur eine Wirkungsergänzung oder -Summierung. Dieser synergistische Effekt tritt besonders stark bei bestimmten Konzentrationsverhältnissen hervor. Im Rahmen der vorliegenden Erfindung hat sich ein Gew.-Verhältnis von bekanntem Wirkstoff zu einer synergistisch wirksamen Verbindung der Formel I von 9:1 bis 1:9, vorzugsweise 4:1 bis 1:4, als günstig erwiesen. Die erfindungsgemässen synergistischen Wirkstoffkombinationen zeichnen sich dadurch aus, dass schon bei geringer Dosierung der angestrebte Effekt eintritt, was vom Standpunkt der Toxikologie und Oekologie einen erheblichen Vorteil darstellt. Von besonderem Interesse erweist sich die Anwendung der erfindungsgemässen synergistischen Wirkstoffkombinationen bei der Bekämpfung resistenter und auch multiresistenter Insektenstämme. Es handelt sich hierbei in der Hauptsache um Resistenz gegenüber Phosphorsäureester. Derartige resistente Stämme konnten mit den bisher üblichen Insektiziden nur schwierig, bzw. nur unter Einsatz bedenklich hoher Wirkstoffdosierungen bekämpft werden.

Unter den Verbindungen der Formel I hat sich das 1-(2,4-Dichlorphenylimino-cyclopropylmethyl)-imidazol als besonders wirksamer Synergist erwiesen.

Die erfindungsgemässen synergistischen Wirkstoffkombinationen weisen bei guter Pflanzenverträglichkeit und geringer Warmblütertoxizität ausgezeichnete Wirksamkeit als Schädlingsbekämpfungsmittel, vor allem als Kontaktinsektizide zur Bekämpfung von Pflanzen und Tiere befallenden Insekten auf.

Die Verbindungen der Formel I enthaltenden synergistischen Wirkstoffkombinationen sind wirksam zur Bekämpfung von Insekten der Familien: Acrididae, Blattidae, Gryllidae,Gryllotalpidae, Tettigoniidae, Cimicidae, Phyrrhocoridae, Reduviidae, Aphididae, Delphacidae, Diaspididae, Pseudococcidae, Chrysomelidae, Coccinellidae, Bruchidae, Scarabaeidae, Dermestidae, Tenebrionidae, Curculionidae, Tineidae, Noctuidae, Lymantriidae, Pyralidae, Galleridae, Culicidae, Tipulidae, Stomoxydae, Muscidae, Calliphoridae, Trypetidae und Pulicidae.

Neben ihrer sehr günstigen Wirkung gegenüber Fliegen, wie z.B. Musca domestica, und Mückenlarven eignen sich die erfindungsgemässen synergistischen Wirkstoffkombinationen insbesondere zur Bekämpfung von pflanzenschädigenden Frassinsekten in Zier- und Nutzpflanzungen, insbesondere in Baumwollkulturen (z.B. Spodoptera littoralis) sowie in Gemüsekulturen (z.B. Myzus persicae).

Die erfindungsgemässen synergistischen Wirkstoffkombinationen sind weiterhin zur Bekämpfung von Ektoparasiten an Haus- und Nutztieren, z.B.,durch Tier-, Stall- und Weideehandlung geeignet.

Die nachfolgenden Beispiele dienen zur näheren Erläuterung der Erfindung. Die Temperaturangaben beziehen sich auf Celsiusgrade, Druckangaben beziehen sich auf Millibar und Teile auf Gewichtsteile.

Herstellungsbeispiele

Beispiel 1: 1-[1-(2,5-Dichlorphenylimino)-2,2-dichlor-äthyl]-imidazol der Formel

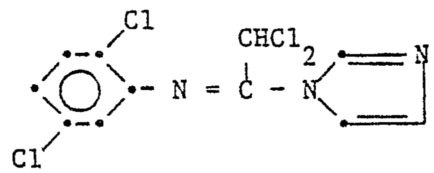

a) Ausgangsmaterial

Die Lösung von 125 g (0,88 Mol) 2,5-Dichloranilin in 1 Liter Chlorbenzol wurde bei 85-80° innerhalb 1 Stunde unter Rühren mit 82 ml (0,85 Mol) Dichloracetylchlorid tropfweise versetzt. Zur Beendigung der HCl-Entwicklung wurde kurz auf 130° erwärmt und anschliessend auf 80° abgekühlt. Hierauf wurden 154 g (0,74 Mol) Phosphorpentachlorid zur Reaktionsmischung hinzugefügt und erneut innerhalb 1 1/2 Stunden auf 130° aufgeheizt. Das Lösungsmittel und das entstandene Phosphoroxidchlorid wurden bei 15 mbar abgedampft und den Rückstand zweimal im Hochvakuum destilliert. Die Ausbeute an N-(1,2,2-Trichloräthyliden)-2,5-dichloranilin vom Sdp.: 96-99°/$10^{-3}$ betrug 118 g.

Auf analoge Weise oder nach einer der hierin beschriebenen Methoden wurden folgende Zwischenprodukte der Formel II hergestellt.

(X=Cl)

| $R_1$ | $(R_2)_n$ | physikalische Konstante |
|---|---|---|
| $-CHCl_2$ | 2,4-$(Cl)_2$ | Kp 90-94° / 0,001 |
| $-CH_2Cl$ | 2,4-$(Cl)_2$ | Kp 90-92° / 0,001 |
| $-CCl_3$ | 2,4-$(Cl)_2$ | Kp 107-111° / 0,01 |
| $-CHCl_2$ | 2,5-$(Cl)_2$ | Kp 96-99° / 0,001 |
| $-CH_2F$ | 2,4-$(Cl)_2$ | Kp 64-66° / 0,005 |
| $-CHClF$ | 2,4-$(Cl)_2$ | Kp 69-72° / 0,001 |
| $-CCl_2F$ | 2,4-$(Cl)_2$ | Kp 76-79° / 0,01 |
| $-CHCl_2$ | - (n=0) | Kp 75-78° / 0,3 |
| $-CCl_3$ | - (n=0) | Kp 136-138° / 16 |
| 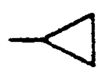 | - (n=0) | |
| 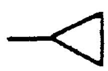 | 4-Br | Kp 98-103° / 0,1 |
| 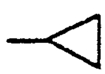 | 2,4-$(Cl)_2$ | Kp 100-105° / 0,3 |
| 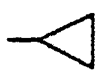 | 3,4-$(Cl)_2$ | Kp 130-135° / 0,1 |
| 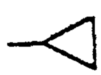 | 4-$OCH_3$ | |
| 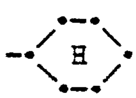 | 4-Br | Kp 132-133° / 0,4 |
| 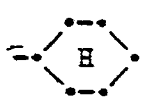 | 3,4-$(Cl)_2$ | Kp 134-140 / 0,15 |
| 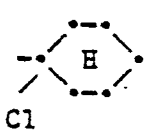 | 4-Br | |
| 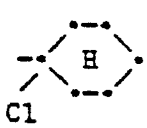 | 3,4$(Cl)_2$ | Kp 158-164° / 0,4 |

| $R_1$ | $(R_2)_n$ | physikalische Konstante |
|---|---|---|
| $-CHCl_2$ | 2-Chlor,-6-Methyl | Kp 84-88° / 0,005 |
| $-CHCl_2$ | $3,5-(Cl)_2$ | Kp 104-108° / 0,005 |
| $-CHCl_2$ | $2,6-(Cl)_2$ | Kp 92-94° / 0,005 |
| $-CH(Cl)(CH_3)_2$ | 4-Br | Kp 93-95° / 0,2 |
| $-CH(Cl)(CH_3)_2$ | $2,4-(Cl)_2$ | Kp 72-75° / 0,15 |
| $-CH(Cl)(CH_3)_2$ | $3,4-(Cl)_2$ | Kp 99-101° / 0,1 |
| $-CH(Cl)(CH_3)_2$ | $4-OCH_3$ | Kp 93-94° / 0,15 |
| $-CH_2Cl$ | $2,6-(CH_3)_2$ | Kp 82-83° / 1 |
| $-CHCl_2$ | $2,6-(CH_3)_2$ | Kp 72-76° / 0,005 |
| $-CCl_3$ | $2,6-(CH_3)_2$ | Kp 82-85° / 0,03 |
| $-CCl_3$ | $2-CH_3$, $6-C_2H_5$ | Kp 94-98° / 0,01 |
| $-CHCl_2$ | $4-NO_2$ | Smp. 44-48° |

b) <u>Endprodukt</u>

Die Lösung von 29,2 g (0,1 Mol) N-(1,2,2-Trichloräthyliden)-2,5-dichloranilin in 50 ml Tetrahydrofuran wurde bei Raumtemperatur unter Rühren innerhalb 20 Minuten zu der Lösung von 15 g (0,22 Mol) Imidazol in 180 ml Tetrahydrofuran hinzugetropft. Nach weiterem dreistündigen Rühren bei Raumtemperatur wurde die Suspension mit 300 ml Wasser und 300 ml Aether verdünnt, die org. Phase mit Wasser neutral gewaschen, getrocknet und eingedampft. Durch Kristallisation aus Isopropyläther wurden 22,5 g 1-[-(2,5-Dichlor-phenylimino)-2,2-dichloräthyl]-imidazol in Form weisser Kristalle erhalten; Smp. 95-97°

Auf analoge Weise oder nach einer der hierin beschriebenen Methoden können folgende Verbindungen der Formel I hergestellt werden.

| Verb.Nr. | $R_1$ | $(R_2)_n$ | physikalische Daten |
|---|---|---|---|
| 1 | $-CHCl_2$ | $2,4-(Cl)_2$ | Smp. 110-112° |
| 2 | $-CH_2Cl$ | $2,4-(Cl)_2$ | |
| 3 | $-CCl_3$ | $2,4-(Cl)_2$ | |
| 4 | $-CHCl_2$ | $2,5-(Cl)_2$ | Smp. 95-97° |
| 5 | $-CH_2F$ | $2,4-(Cl)_2$ | Smp. 40-44° |
| 6 | $-CHClF$ | $2,4-(Cl)_2$ | Smp. 88-90° |
| 7 | $-CCl_2F$ | $2,4-(Cl)_2$ | Sdp. 110-115°/0.001 Torr. |
| 8 | $-CHCl_2$ | - (n=0) | Smp. 82° |
| 9 | $-CCl_3$ | - (n=0) | |
| 10 | $-CHCl_2$ | 2-Cl, 6-$CH_3$ | Smp. 111-114° |
| 11 | $-CHCl_2$ | $3,5-(Cl)_2$ | Smp. 100-102° |
| 12 | $-CHCl_2$ | $2,6-(Cl)_2$ | Smp. 145-147° |
| 13 | $-CCl(CH_3)_2$ | $2,4-(Cl)_2$ | honigartig , $n_D^{28}$ = 1,5255 |
| 14 | $-CCl(CH_3)_2$ | $3,4-(Cl)_2$ | Smp. 79-81° |
| 15 | $-CCl(CH_3)_2$ | $4-CH_3O$ | Smp. 110-113° |
| 16 | $-CCl(CH_3)_2$ | 4-Br | Smp. 90-91° |
| 17 | 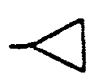 | - (n=0) | |
| 18 | 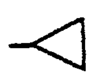 | 4-Br | |
| 19 | 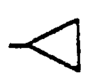 | $2,4-(Cl)_2$ | Smp. 66-67° |
| 20 | 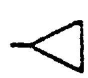 | $3,4-(Cl)_2$ | Smp. 83-85° |
| 21 | 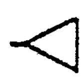 | $4-OCH_3$ | |

| Verb.Nr. | $R_1$ | $(R_2)_n$ | physikalische Daten |
|---|---|---|---|
| 22 | 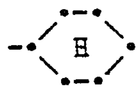 | 4-Br | |
| 23 | 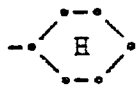 | $3,4-(Cl)_2$ | |
| 24 | 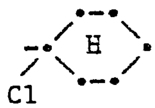 | 4-Br | |
| 25 | 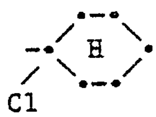 | $3,4-(Cl)_2$ | honigartig , Smp.54-59° |
| 26 | $-CHCl_2$ | 4-Cl | |
| 27 | $-CH_2F$ | 4-Cl | |
| 28 | $-CHCl_2$ | 4-Br | Smp. 119-122° |
| 29 | $-CH_2F$ | 4-Br | |
| 30 | $-CHCl_2$ | $4-(CH_3)_3C-$ | |
| 31 | $-CHCl_2$ | $3-CF_3$, 4-Cl | |
| 32 | $-CHCl_2$ | $2-CH_3O$, 4-Cl | |
| 33 | $-CH_2F$ | $2-CH_3O$, 4-Cl | |
| 34 | $-CHCl_2$ | $2-CH_3$, 4-Cl | |
| 35 | $-CH_2F$ | $2-CH_3$, 4-Cl | |
| 36 | $-CCl_3$ | $3-NO_2$ | |
| 37 | $-CH_2Cl$ | $2,6-(CH_3)_2$ | |
| 38 | $-CHCl_2$ | $2,6-(CH_3)_2$ | Smp: 85°-88° |
| 39 | $-CCl_3$ | $2,6-(CH_3)_2$ | |
| 40 | $-CCl_3$ | $2-CH_3$, $6-C_2H_5$ | |
| 41 | $-CHCl_2$ | $4-NO_2$ | Smp. 200° (Zers.) |

| Verb.Nr. | $R_1$ | $(R_2)_n$ | physikalische Daten |
|---|---|---|---|
| 42 | $-CHCl_2$ | - (n = 0) | Smp. 82° |
| 43 | $-CHCl_2$ | $3,4-(Cl)_2$ | Smp. 116-119° |
| 44 | $-CCl\ (CH_3)_2$ | - (n = 0) | Smp. 82-83° |
| 45 | $-CCl\ (CH_3)_2$ | $4-CH_3$ | Smp. 87-88° |
| 46 | $-CCl\ (CH_3)_2$ | $4-Cl,\ 3-CF_3$ | Smp. 67-68° |
| 47 | 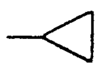 | $4-Cl,\ 3-CF_3$ | Smp. 92-94° |
| 48 | 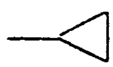 | $4-Cl,\ 2-CH_3$ | $n_D^{28}$ = 1,6055 |
| 49 | 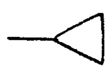 | $3-CF_3$ | $n_D^{28}$ = 1,5465 |
| 50 | 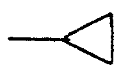 | $3,5-(CF_3)_2$ | Smp. 91-93° |

## Formulierungsbeispiele

### Beispiel 2:

Stäubemittel: Zur Herstellung eines a) 5 %igen, b) 2 %igen und c) 80 %igen Stäubemittels werden die folgenden Stoffe verwendet:

a) 5 Teile Wirkstoff
95 Teile Talkum;

b) 2 Teile Wirkstoff
1 Teil hochdisperse Kieselsäure,
97 Teile Talkum;

c) 80 Teile Wirkstoff
17 Teile Talkum
3 Teile hochdisperse Kieselsäure

Die Wirkstoffe werden mit den Trägerstoffen vermischt und vermahlen und können in dieser Form zur Anwendung verstäubt werden.

### Beispiel 3:

Granulat: Zur Herstellung eines 5 %igen Granulates werden die folgenden Stoffe verwendet:

5 Teile Wirkstoff
0,25 Teile epoxydiertes Pflanzenöl,
0,25 Teile Cetylpolyglykoläther,
3,50 Teile Polyäthylenglykol
91 Teile Kaolin (Korngrösse 0,3 - 0,8 mm).

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 4:

Spritzpulver: Zur Herstellung eines a) 70 %igen b) 40 %igen c) und d) 25 %igen e) 10 %igen Spritzpulvers werden folgende Bestandteile verwendet:

a)
70 Teile Wirkstoff
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin
12 Teile Champagne-Kreide;

b)
40 Teile Wirkstoff
5 Teile Lingninsulfonsäure-Natriumsalz,
1 Teil Dibutylnaphthalinsulfonsäure-Natriumsalz,
54 Teile Kieselsäure;

c)
25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d)
25 Teile Wirkstoff
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff
3 Teile Gemisch der Natriumsalze von gesättigten Fettalkoholsulfaten,
5 Teile Naphthalinsulfonsäure/Formaldehyd-Kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 5:

Emulgierbare Konzentrate: Zur Herstellung eines 25 %igen emulgierbaren Konzentrates werden folgende Stoffe verwendet:

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Anwendungskonzentration hergestellt werden, die besonders zur Blattapplikation geeignet sind.

Biologische Beispiele

Beispiel 6:

Wirkung gegen Erysiphe graminis auf Gerste

Ca. 8 cm hohe Gerstenpflanzen wurden mit einer aus Spritzpulver des Wirkstoffes hergestellten Spritzbrühe (0,02 % Aktivsubstanz) besprüht. Nach 4 Stunden wurden die behandelten Pflanzen mit Konidien

des Pilzes bestäubt. Die infizierten Gerstenpflanzen wurden in einem Gewächshaus bei ca. 22°C aufgestellt und der Pilzbefall nach 10 Tagen beurteilt.

Beispiel 7:

Wirkung gegen Piccinia graminis auf Weizen

Weizenpflanzen wurden 6 Tage nach der Aussaat mit einer aus Spritzpulver des Wirkstoffes hergestellten Spritzbrühe (0,06 %) Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20°C wurden die infizierten Pflanzen in einem Gewächshaus bei ca. 22°C aufgestellt.

Die Beurteilung der Rostpustelnentwicklung erfolgte 12 Tage nach der Infektion.

Beispiel 8:

Wirkung gegen Tilletia caries

Tilletiasporen werden in einer Spritzbrühe enthaltend 600 ppm Wirkstoff während 15 min. suspendiert. Das Sporen-Substanz-Gemisch wird tropfweise auf die Oberfläche von fein gesiebter, angefeuchteter Erde in Petrischalen pipettiert. Die so zubereiteten Erdschalen werden bei hoher Luftfeuchtigkeit und einer Temperatur von 20°C aufgestellt. Nach ca. 10 Tagen wird die Sporenkeimung unter der Lupe beurteilt. Die Wirkung der Testsubstanzen wird auf Grund der Anzahl und der Länge der Keimschläuche ermittelt.

Verbindungen gemäss Beispiel 1 zeigten in den obigen Versuchen (Beispiele 6, 7 und 8) gute Wirkung.

Unter anderem zeigten nachstehend genannte Verbindungen der Formel I bei folgenden Pilzen eine Hemmung des Befalls auf weniger als 20 % verglichen mit unbehandelten Kontrollpflanzen:

| | |
|---|---|
| Bei Erysiphe graminis: | 1, 8, 10, 13, 14 und 25 |
| Bei Puccinia graminis: | 4, 6, 8, 11 und 13 |
| Bei Tilletia caries: | 4 und 8. |

Beispiel 9:

Wirkung gegen Xanthomonas vesicatoria auf Paprika

a) Residual-protektive Wirkung

Paprikapflanzen der Sorte "California Wonder" wurden nach 3-wöchiger Anzucht im Gewächshaus mit der Prüfsubstanz in Form einer Spritzbrühe (0,06 % Aktivsubstanz) besprüht. Nach eintägigem Antrocknen dieses Spritzbelages wurden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der residualen Wirksamkeit der Prüfsubstanz.

b) Systemische Wirkung

Paprikapflanzen der Sorte "California Wonder" wurden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung wurden die Pflanzen in einem Klimaraum bei 20°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige,später nektrotische, aufgehellte Flecken. Das Ausmass dieser Flecken diente zur Beurteilung der systemischen Wirksamkeit der Prüfsubstanz.

Verbindungen gemäss Beispiel 1 zeigten gute Wirkung in obigen Versuchen.

In den obigen Versuchen zeigten besonders Verbindungen Nr. 5 und 41 der Formel I eine starke bakterizide Wirkung.

Beispiel 10:

Akarizide Wirkung: Tetranychus urticae (OP-sensible) und Tetranychus cinnabarinus (OP-tolerant)

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit gegenüber Diazinon.) Die so behandelten, durch die übergelaufenen beweglichen Individuen infestierten Pflanzen wurden mit einer Versuchslösung enthaltend 400, 200 oder 100 ppm der erfindungsgemässen Verbindung bis zur Tropfnässe besprüht.

Nach 24 Stunden und wiederum nach 7 Tagen wurde unter dem Binokular auf lebende und tote Individuen ausgewertet.

Man verwendete pro Konzentration und pro Testspezies eine Pflanze. Während des Versuchsverlaufs standen die Pflanzen in Gewächskabinen bei 25°C.

Verbindungen gemäss Beispiel 1 zeigten gute Wirkung in obigem Versuch.

Unter anderem zeigten die Verbindungen 5 und 6 im obigen Versuch eine gute Wirkung gegen Adulte, Larven und Eier der Spezies Tetranychus urticae und Tetranychus cinnabarinus.

Beispiel 11:

Synergistische Insektizidwirkung auf resistente Stämme von Musca domestica und Spodoptera littoralis:

a) Es wurden adulte Stubenfliegen (Musca domestica), die gegen insektizide Phosphorsäureester Resistenz entwickelt hatten, durch Kälteschock inaktiviert. Dann wurde mittels eines Mikro-Applikators 1 µl der jeweiligen Wirkstofflösung (Lösungsmittel Aceton/Wasser: 80/20) auf die Oberseite des Abdomens eines jeden Versuchstieres aufgebracht. Es wurde die synergistische Wirkung der erfindungsgemässen Verbindung 1-(2,4-Dichlorphenylimino-cyclopropylmethyl)-imidazol, nachstehend als "Synergist" bezeichnet, in Bezug auf das bekannte Insektizid "Nuvacron" [0,0-Dimethyl-0-(2-methylcarbamoyl-1-methyl-vinyl)-phosphat] geprüft.

Die folgenden Dosierungen, d.h. applizierten Mengen an Wirkstoffen pro Versuchstier, wurden verwendet:

1.) 5 µg bzw. 10 µg "Nuvacron" (ohne "Synergist"-Zusatz)

2.) 5 µg bzw. 10 µg "Synergist" (ohne "Nuvacron"-Zusatz)

3.) 5 µg "Nuvacron" + 5 µg "Synergist" bzw. 10 µg "Nuvacron" + 10 µg Synergist.

Nach 24 Stunden wurde die prozentuale Mortalität der behandelten Fliegen bestimmt. Die unter 3.) aufgeführten synergistischen Gemische zeigten erheblich höhere Abtötungsraten als die unter 1.) und 2.) aufgeführten Einzelkomponenten.

b) Mit Hilfe eines Mikro-Applikators wurden auf Larven von Spodoptera littoralis im vierten Larvalstadium, die gegen insektizide Phosphoräsureester resistent waren, pro Versuchstier 2 µl der jeweiligen Wirkstofflösung (Lösungsmittel Aceton/Wasser: 80/20) topikal appliziert.

Es wurde die synergistische Wirkung der erfindungsgemässen Verbindung 1-(2,4-Dichlorphenylimino-cyclopropylmethyl)-imidazol, nachstehend als "Synergist" bezeichnet, in Bezug auf das bekannte Insektizid "Nuvacron" [O,O-Dimethyl-O-(2-methylcarbamoyl-1-methylvinyl-phosphat] geprüft.

Die folgenden Dosierungen, d.h. applizierten Mengen an Wirkstoffen pro Versuchstier, wurden verwendet:

1.) 4 µg "Nuvacron" (ohne "Synergist"-Zusatz)
2.) 4 µg "Synergist" (ohne "Nuvacron"-Zusatz)
3.) 4 µg "Nuvacron" + 4 µg "Synergist".

Nach 24 Stunden wurde die prozentuale Mortalität der behandelten Larven bestimmt. Das unter 3.) aufgeführte synergistische Gemisch zeigte eine erheblich höhere %-Abtötung als die unter 1.) und 2.) aufgeführten Einzelkomponenten.

Patentansprüche

1. Verbindung der Formel I

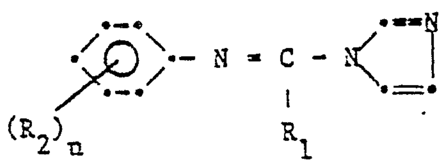

(I)

worin n für eine ganze Zahl zwischen 0 und 4 steht, $R_1$ $C_1$-$C_6$-Halogenalkyl, $C_3$-$C_6$ Cycloalkyl oder $C_3$-$C_6$ Halogencycloalkyl bedeutet, und $R_2$ eine durch n bestimmte Zahl gleicher oder verschiedener Substituenten aus der Gruppe Halogen, $C_1$-$C_4$-Alkyl, $C_1$-$C_4$-Alkoxy, Nitro, Trifluormethyl oder Cyano bedeutet.

2. Verbindung der Formel I gemäss Anspruch 1, dadurch gekennzeichnet, dass

$R_1$ durch 1-3 Halogenatome substituiertes Methyl, $C_3$-$C_6$-Cycloalkyl oder durch 1 Chloratom substituiertes $C_3$-$C_6$ Cycloalkyl und

$R_2$ Chlor, Brom, Methoxy, Methyl, Aethyl oder Trifluormethyl bedeuten.

3. Verbindung der Formel I gemäss Anspruch 2, dadurch gekennzeichnet, dass

$R_1$ $C_3$-$C_6$-Cycloalkyl oder durch 1 Chloratom substituiertes Cyclohexyl und

$R_2$ Chlor, Methoxy oder Trifluormethyl bedeuten.

4. Verbindung der Formel I gemäss Anspruch 3, dadurch gekennzeichnet, dass

$R_1$ $C_3$-$C_6$-Cycloalkyl und

$R_2$ Chlor

bedeuten.

5. Verbindung der Formel I gemäss einem der Ansprüche 1 bis 4, dadurch gekennzeichnet, dass n für eine ganze Zahl zwischen 0 und 2, vorzugsweise für 1 oder 2, steht.

6. Verbindung der Formel I gemäss Anspruch 4, dadurch gekennzeichnet, dass

n für 2 steht,

$R_1$ Cyclopropyl und

$R_2$ Chlor bedeuten.

7. Verbindung gemäss Anspruch 6 der Formel

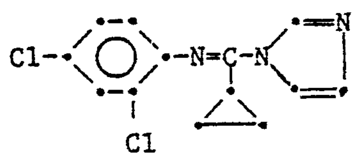

.

8. Verbindung gemäss Anspruch 2 der Formel

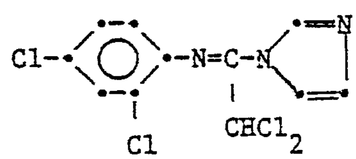

.

9. Verfahren zur Herstellung einer Verbindung der Formel I gemäss Anspruch 1, dadurch gekennzeichnet, dass man eine Verbindung der Formel II

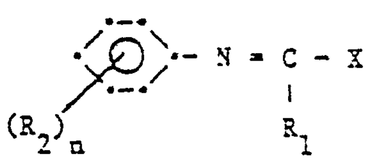

(II)

mit einer Verbindung der Formel III

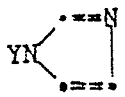

(III)

umsetzt, wobei in den Formeln II und III $R_1$, $R_2$ und n die für Formel I angegebenen Bedeutungen haben, X für ein Nukleofug und Y für Wasserstoff oder ein Metallatom stehen.

10. Schädlingsbekämpfungsmittel enthaltend als aktive Komponente mindestens eine Verbindung gemäss Anspruch 1 bis 8.

11. Mittel gemäss Anspruch 10 zur Bekämpfung von phytopathogenen Mikroorganismen und Vertretern der Ordnung Akarina.

12. Mittel gemäss Anspruch 10, welches als synergistisch aktive Komponente eine Verbindung gemäss den Ansprüchen 1 bis 8 zusammen mit einem pestizid wirksamen Phosphorsäureester, Chlorkohlenwasserstoff, Carbamat oder Pyrethroid enthält.

13. Mittel gemäss Anspruch 12 zur Bekämpfung von Insekten, welches als synergistisch aktive Komponente eine Verbindung gemäss den Ansprüchen 1 bis 8 zusammen mit einem pestizid wirksamen Phosphorsäurester enthält.

14. Mittel gemäss Anspruch 13, welches als synergistisch aktive Komponente die Verbindung gemäss Anspruch 7 enthält.

15. Verwendung einer Verbindung gemäss den Ansprüchen 1 bis 8 zur Bekämpfung von Schädlingen.

0019581

Europäisches Patentamt

# EUROPÄISCHER RECHERCHENBERICHT

Nummer der Anmeldung
EP 80 81 0138

| EINSCHLÄGIGE DOKUMENTE | | | KLASSIFIKATION DER ANMELDUNG (Int Cl) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, Band 87, Nr. 15, 10. Oktober 1977, Seite 600, Nr. 117860t<br>Columbus, Ohio, U.S.A.<br>& JP - A - 77 46071 (NIPPON SODA CO., LTD.) 12-04-1977<br>* Zusammenfassung *<br>-- | 1,10 | C 07 D 233/61<br>A 01 N 43/50<br>C 07 C 119/16 |
| D,A | CHEMICAL ABSTRACTS, Band 87, Nr. 19, 7. November 1977, Seite 596, Nr. 152199f<br>Columbus, Ohio, U.S.A.<br>& JP - A - 77 39674 (NIPPON SODA CO., LTD.) 28-03-1977<br>* Zusammenfassung *<br>-- | 1,10 | RECHERCHIERTE SACHGEBIETE (Int. Cl)<br>C 07 D 233/61 |
| D,A | BE - A - 2 814 041 (NIPPON SODA)<br>* Seiten 4-8 *<br>---- | 1,10 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 04-08-1980 | DE BUYSER |